# EUROPEAN PATENT APPLICATION

(11) **EP 4 635 459 A2**
(43) Date of publication of application: **22.10.2025**
(21) Application number: 25162350.0
(22) Date of filing: 07.03.2025
(51) Int. Cl.: A61F 7/00, A61G 11/00

(54) **SYSTEMS AND METHODS FOR HEATING AN INFANT CARE STATION**

(30) Priority: 29.03.2024 US 202418622509
(71) Applicant: GE Precision Healthcare LLC, Waukesha, WI 53188 (US)
(72) Inventor: NAIK, Rajendra, 560066 Bengaluru (IN); DHULIPALLA, Ravi Kumar, 560066 Bengaluru (IN); KLOCKOW, Helge B., Wauwatosa, 53226 (US); SEARLES, Gabriel M., Madison, 53718 (US)
(74) Representative: Kilburn & Strode LLP

(57) **Abstract**

An infant care station is described herein that includes a support platform for housing an infant and a heater for generating radiant heat to be provided to the support platform. In some examples, a reflective dish of the heater includes one or more vents to allow hot air proximate to a heater element of the heater to flow through a back of the reflective dish. The hot air can include impurities from air and the heater can provide radiant heat to the support platform as the hot air with the impurities flows through the back of the reflective dish.

## Description

### FIELD

Techniques disclosed herein relate to an infant care station, and more particularly to heating an infant care station with a vented heater.

### BACKGROUND

Some infant patients are not physiologically well enough developed to be able to survive without special medical attention. A frequently used medical aid for such infants is the incubator. The primary objective of the incubator is to provide an environment which will maintain the infant at a minimum metabolic state thereby permitting as rapid physiological development as possible. Neonatal incubators create a microenvironment that is thermally neutral where an infant can develop. These incubators typically include a humidifier and a heater and associated control system that controls the humidity and temperature in the neonatal microenvironment. The humidifier comprises a device that evaporates an evaporant, such as distilled water, to increase relative humidity of air within the neonatal microenvironment. The humidifier is typically controllable such that the amount of water, or water vapor, added to the microenvironment is adjustable in order to control the humidity to a desired value. The heater may be, for example, an air heater controllable to maintain the microenvironment area to a certain temperature. In some examples, radiant warmers may be used instead of incubators for some infants where less environmental control is required. In still other embodiments, hybrid incubator/radiant warming systems may be utilized.

Since the microenvironment is accurately controlled in a neonatal or infant care station, the infant care station includes an enclosure that is sealed to help maintain the controlled microenvironment. Such an enclosure, also called the infant compartment, will typically include four sidewalls or side panels and a top hood or canopy that surround an infant support platform.

Infant care stations can use any number of heaters to provide warm air to an infant residing on an infant support platform such as a mattress or a support platform of the infant care station. In some examples described herein, an infant care station can include a heater that can have one or more vents in a reflective dish, wherein the vents allow hot air with impurities to pass through the reflective dish of the heater while the heater provides radiant heat to a support platform.

### BRIEF DESCRIPTION

In one example, an infant care station is described herein that includes a support platform for housing an infant and a heater for generating radiant heat to be provided to the support platform. In some examples, a reflective dish of the heater includes one or more vents to allow hot air proximate to a heater element of the heater to flow through a back of the reflective dish. The hot air can include impurities and the heater can provide radiant heat to the support platform as the hot air with the impurities flows through and out the back of the reflective dish.

In another example, a method for operating an infant care station can include determining an expected amount of impurities to be removed from air. The method can also include determining a vent configuration for the heater based on the expected amount of impurities and modifying one or more settings for the heater based on the vent configuration. The method can also include providing radiant heat to a support platform of the infant care station using the one or more settings while enabling hot air to flow through one or more vents in the reflective dish of the heater.

In yet another example, a non-transitory computer-readable medium for operating an infant care station with a vented heater can include a plurality of instructions that in response to execution by a processor, cause the processor to determine an expected amount of impurities to be removed from air. The plurality of instructions can also cause the processor to determine a vent configuration for the heater based on the expected amount of impurities and modify one or more settings for the heater based on the vent configuration. The plurality of instructions can also cause the processor to provide radiant heat to a support platform of the infant care station using the one or more settings while enabling hot air to flow through one or more vents in the reflective dish of the heater.

### BRIEF DESCRIPTION OF THE DRAWINGS

The present disclosure will be better understood from reading the following description of non-limiting examples, with reference to the attached drawings broadly described below:
FIG. 1 is a perspective view of an example infant care station operating as an infant warmer with a heater and a canopy in accordance with one example;
FIG. 2 is an example infant care station using a heater to provide radiant heat in an infant warmer setting without a canopy in accordance with one example;
FIG. 3A shows a front view of an example heater with vents for an infant care station in accordance with one example;
FIG. 3B depicts a vented heater in accordance with one example;
FIG. 4 depicts air flow in a heater without central vents;
FIG. 5 shows an example air flow through a heater with vents for an infant care station in accordance with examples;
FIG. 6 shows a process flow diagram of an example method for operating a heater with vents for an infant care station in accordance with examples;
FIG. 7 shows a back view of an example heater with one or more vents in accordance with examples;
FIGS. 8A and 8B depict a back view of example heaters with one or more ducts coupled to vents of the heater in accordance with examples;
FIG. 9 is a block diagram of an example of a computing device that can operate a heater with vents in an infant care station; and
FIG. 10 is an example of a non-transitory machine-readable medium for operating a heater with vents in an infant care station, in accordance with examples.

The drawings illustrate specific aspects of the described components, systems and methods for providing a neonatal infant care station. Together with the following description, the drawings demonstrate and explain the principles of the structures, methods, and principles described herein. In the drawings, the thickness and size of components may be exaggerated or otherwise modified for clarity. Well-known structures, materials, or operations are not shown or described detail to avoid obscuring aspects of the described components, systems and methods.

### DETAILED DESCRIPTION

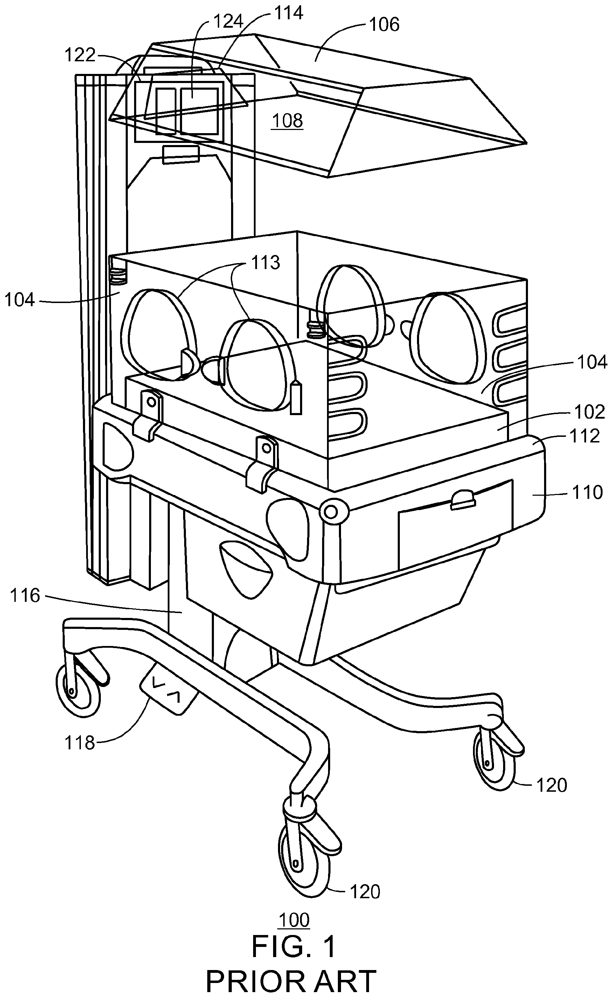

Embodiments of the present disclosure will now be described, by way of example, with reference to FIGS. 1-10, in which the following description relates to various examples of infant care stations. The following description relates to various techniques, methods, non-transitory computer-readable media, and systems for heating an infant care station with one or more vented heaters. A vented heater, as referred to herein, can include any suitable heater to generate radiant heat to be provided to an infant care station while also enabling some hot air to pass through one or more vents in a reflective dish of the vented heater. Infant care stations can provide microenvironments for infant patients receiving medical care. Infant care stations, as referred to herein, can include incubators, warmers, or devices that support one or more features of incubators and warmers. The infant care stations can use one or more vented heaters to provide radiant heat to an infant residing on a support platform, mattress, bed or the like of the infant care station.

Techniques herein have the technical advantage of minimizing or eliminating the reduction in reflective efficiency of the radiant heaters over the lifetime of infant care stations by configuring reflective dishes of the heaters to include vents. The vents can be placed in the reflective dishes at any number of locations that enable hot air with impurities to flow through to the back of the reflective dish and away from the infant care station. The vents can enable impurities from air to be removed from the air above a support platform.

FIG. 1 is a perspective view of an example infant care station operating as an infant warmer with a heater and a canopy. In some examples, the infant care station 100 can include a horizontal surface 102, walls 104, and canopy 106 being connected. In some examples, the horizontal surface 102, walls 104, and canopy 106 may be individual components that also may be moveable with respect to each other. For example, the example configuration of FIG. 1 depicts an open canopy 106 raised away from the walls 104 to allow operation as a warmer in which the microenvironment is exposed to the surrounding environment of the infant care station 100.

The horizontal surface 102, walls 104, and canopy 106 can define a microenvironment 108 that is exposed to air due to the raised canopy 106. In some examples, the walls 104 further include arm portholes 113 that permit a clinician access into the microenvironment 108. In some examples, the walls 104 can be shorter or taller and may not include arm portholes 113 in a warmer configuration.

In some examples, the infant care station 100 includes a base 110 that supports a support platform 112. The infant care station 100 can also include a radiant heater 114 above the support platform 112. The radiant heater 114 can be configured to provide radiant heat to the support platform 112 to warm an infant patient. The radiant heater 114 in FIG. 1 is a dedicated heater for providing radiant heating without the ability to modulate or provide convective heating. In some examples, the infant care station 100 may include a separate second heater (not depicted) to provide thermally conditioned air in an incubator setting with a closed canopy 106.

Examples of the infant care station 100 further include a pedestal 116 connected to the base 110. The pedestal 116 includes mechanical components (not depicted), which may include, but are not limited to, servo motors, rack and pinion systems, or screw gear mechanisms that are operable by foot pedals 118 to raise or lower the base 110, effectively raising or lowering the position of the infant patient (not depicted) in relation to the clinician. The infant care station 100 may be moveable by wheels or casters 120 connected to the pedestal 116.

The example of the infant care station 100 depicted in FIG. 1 includes a graphical display 122 that is mounted to a wall, the base 110, or the canopy 106 of the infant care station 100 at a position external to the microenvironment 108. The graphical display 122 is operated by a processor to present a graphical user interface (GUI) 124. In the example illustrated, the graphical display 122 is a touch-sensitive graphical display and the GUI 124 is configured to specifically respond to inputs made by a clinician received through the touch-sensitive graphical display. During normal operation, the touch-sensitive graphical display 122 and touch-sensitive configured GUI 124 are used to control various functions of the infant care station 100. The GUI 124 presents a variety of information, such as the air temperature and alarm indications. In some examples, the alarm indications can provide a message indicating a change in environment characteristics, or a warning that an infant patient is too warm, among others.

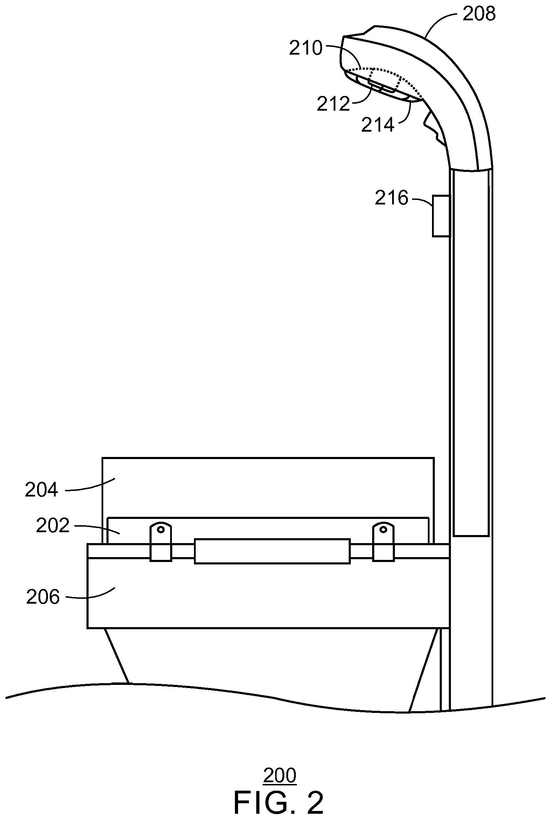

FIG. 2 is an example infant care station using a heater to provide radiant heat in an infant warmer setting without a canopy in accordance with one example. In some examples, the infant care station 200 can include a horizontal surface 202 and walls 204. In some examples, the support platform or horizontal surface 202 and walls 204 may be individual components that also may be moveable with respect to each other. For example, the example configuration of FIG. 2 depicts an open microenvironment without a canopy above the walls 204 to allow operation as an infant warmer in which the microenvironment is exposed to the surrounding air and environment of the infant care station 200.

In some examples, the infant care station 200 includes a base 206 that supports a horizontal surface or support platform 202. The infant care station 200 can also include a housing 208 that can include any number of components such as a heater 210 proximate to the support platform 202. The heater 210 can be configured to provide radiant heat to the support platform 202 in a uniform manner to warm an infant patient. For example, the heater 210 can include a heater element 212 and a reflective dish 214. The reflective dish 214 can enable radiant heat to be provided to an infant patient on the horizontal surface 202. In some examples, the heater 210 provides radiant heat using the reflective dish 214 or any suitable reflective surface to enable a radiant heating setting for the infant care station 200 in order to provide radiant heat to maintain a predetermined temperature of the infant patient.

The heater element 212 and the reflective dish 214 can be configured to project the generated radiant heat using any number of focal points. For example, the reflective dish 214 can be parabolic, elliptical, or any other suitable geometric shape to provide radiant heat.

In some examples, the reflective dish 214 can be located over a mattress or support platform 202 of the infant care station 200, over a display panel 216 of an infant care station 200, behind a display panel 216 of the infant care station 200, or any other suitable location proximate to the support platform 202 or mattress.

It is to be understood that the block diagram of FIG. 2 is not intended to indicate that the infant care station 200 is to include all of the components shown in FIG. 2. Rather, the infant care station 200 can include fewer or additional components not illustrated in FIG. 2 (e.g., additional walls, porthole access points, display panels, or drawers, etc.). For example, the infant care station 200 can include any number of heaters 210 that can provide, generate, or otherwise emit radiant heat toward the support platform or horizontal surface 202 of the infant care station 200. In some examples, two or more heaters 210 can be positioned proximate one another and can simultaneously modulate to provide convective heating for an incubator setting or radiant heating for an infant warmer setting.

In some examples, one or more heaters 210 can be configured to accept or utilize multiple heater elements 212. For example, a heater 210 may include two or more heater elements 212 that emit or generate radiant heat that warms air within the infant care station 200 by projecting radiant heat onto the reflective dish 214. In some examples, the heater elements 212 of the heater 210 can be made of the same materials or different materials and the heater elements 212 may be configured to generate the same amount of radiant heat or different amounts of radiant heat.

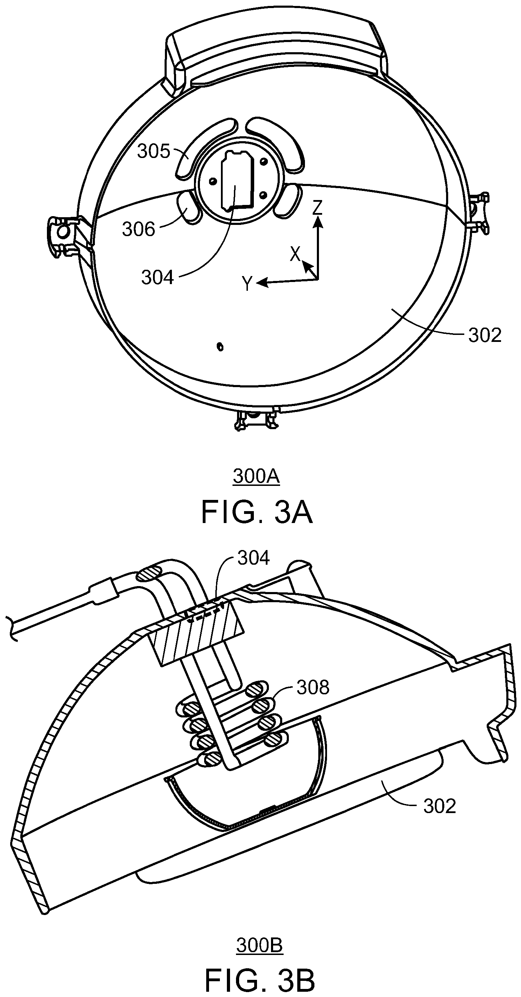

FIG. 3A shows a front view of an example heater with vents for an infant care station in accordance with one example. In some examples, a reflective dish 302 of a heater 300A can include any number of vents 304. The vents 304 can allow air to flow through the reflective dish 302 rather than being circulated proximate to and beneath the heater 300A. In some examples, the number of vents 304 can be determined based on any number of characteristics such as an amount of air flow around a heater element (308 of FIG 3B) coupled to the reflective dish 302, a temperature variability on a support platform based on radiant heat from the reflective dish 302, and a flow of air through vents 304 and behind the reflective dish 302, among others.

In some examples, a size and shape of each vent 304 can also be determined based on the predetermined characteristics. The example of FIG. 3A illustrates a reflective dish 302 that includes two larger vents 305 and two smaller vents 306. In some examples, the positioning of the vents 304 can be within a predetermined distance from the heater element (308 of FIG. 3B) or center of the reflective dish 302. For example, the vents 304 can be located proximate to the center of the reflective dish 302 so that a portion of the reflective dish 302 along the outer edge or perimeter maintains a solid surface.

The vented heater 300A illustrated in FIG. 3A includes a number of vents 304 that are cut out or otherwise manufactured in a reflective dish 302 such that the hot air or radiant heat rising from the heater element area is vented out, without touching the surface of the reflective dish 302. The techniques herein have the technical advantage of preventing any deposition of material on the reflective dish 302 and reflectivity is not compromised even over time.

The reflective dish is 302 can be shaped such that much of the radiant heat from the heater element (308 of FIG. 3B) is reflected onto the support platform providing uniform radiant heating. Because of the design of the reflective dish 302, there are certain areas on the reflective dish 302 that do not focus the radiant heat onto the support platform, so any vent 304 there does not contribute to providing radiant heat. In some examples, as the position and the size of the vent 304 varies, the reflective surface varies and therefore the amount of radiant heat projected onto the support platform changes. This can lead to temperature changes for the same power/heat setting on the heater 300A. With a larger vent 305, and/or a vent placed in a portion of the reflective dish 302 that provides radiant heat to the support platform, the loss will be more, leading to running the heater at a higher power to get the same amount of radiant heat onto the support platform.

In some examples, larger vents 305 can lead to minimal deposits of impurities on the reflective surface and therefore any change in reflectivity over time is below a predetermined threshold. In some examples, a larger vent 305 can also result in a loss of a substantial area of the reflective surface, and the heater 300A has to be driven harder to provide the same amount of heat onto the support platform from other remaining surfaces of the reflective dish 302.

FIG. 3B depicts a vented heater 300B in accordance with one example. The vented heater 300B can include one or more heater elements 308 that provide heat to be reflected by the reflective dish 302. In some examples, hot air from the heater element 308 can flow through the vent 304 while radiant heat is projected downward towards a support platform of an infant care station.

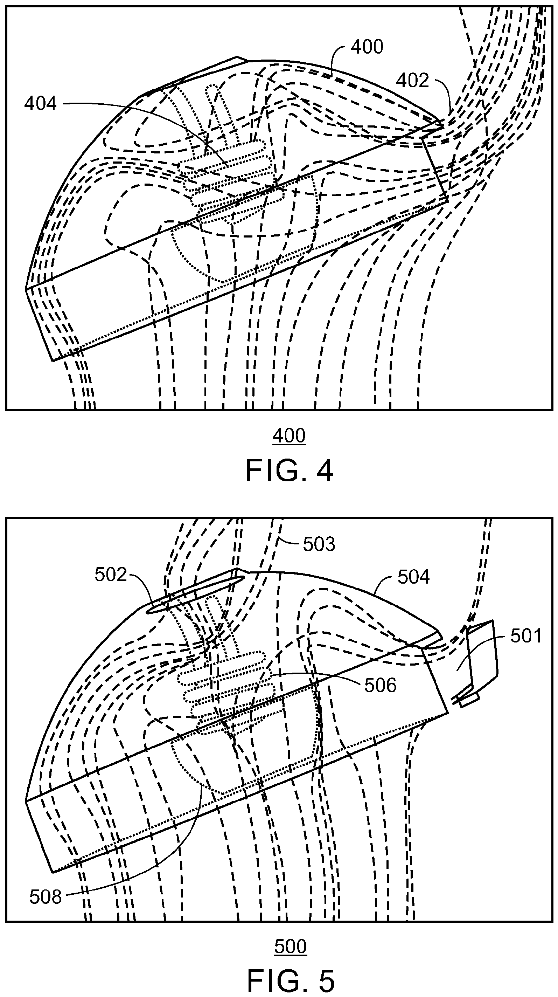

FIG. 4 depicts air flow in a heater without central vents. The vent 402 along the perimeter of reflective dish 400 enables hot air to flow away from the infant care station. However, impurities from the air now circulate over the surface of the reflective dish 400 and the heater element 404 and there is a higher possibility of deposition of these impurities on the reflective surface, thereby compromising the reflectivity over time.

FIG. 5 shows an example air flow through a heater with vents for an infant care station in accordance with examples. The vented heater 500 of FIG 5 can include any number of vents 501 and 502 that enable hot air 503 with impurities to flow or pass through to the back of the reflective dish 504. For example, any number of centrally located vents 502 can be placed proximate to a heater element 506 coupled to the reflective dish 504. The heater element 506 can project radiant heat below to a support platform while any impurities in hot air circulating the heater element 506 can flow through the vents 501 and 502.

In some examples, the vented heater 500 is placed at an angle over a support platform of an infant care station. Further, there can be a deflector cup 508 that blocks the radiant heat generated by the vented heater 500 from certain portions of the reflective dish 504 from reaching the support platform. In some examples, any changes in the vented portions of the reflective dish 504 may not affect the irradiance and uniformity of the radiant heat that is provided to the support platform from the vented heater 500.

In some examples, the location, shape, contour and size of the cutouts or vents 502 of the reflective dish 504 can be determined. Further, by modeling the air flow, the contours can be optimized to channel the hot air out of the enclosed surface while minimizing the loss of radiant heat reflected onto the infant platform. In addition, a heater element 506 of the vented heater 500 can be pre-oxidized to form a barrier oxide coating that also reduces the volatilization of oxides from the vented heater 500. In some examples, the heater element 506 can produce the same amount of radiant heat but at a proportionately lower operating temperature, which can further enhance the life of the heater element 506. In some examples, the vented heater 500 can rotate to face downward in the infant care station to provide radiant heat to a heat sink or any other suitable components that distribute thermally conditioned air under the support platform.

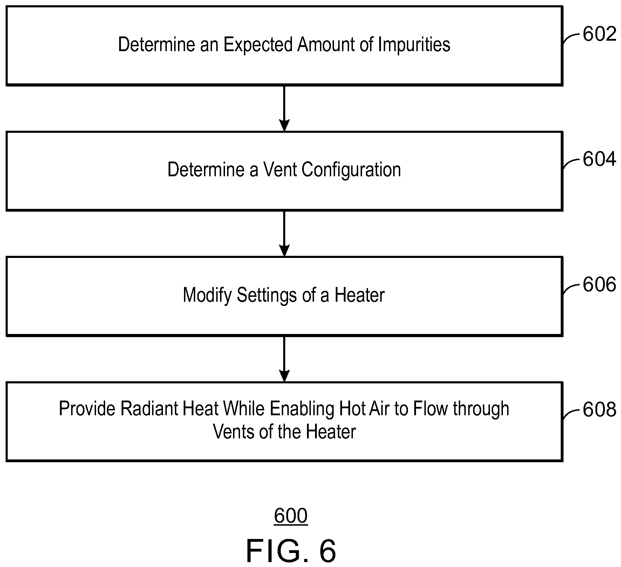

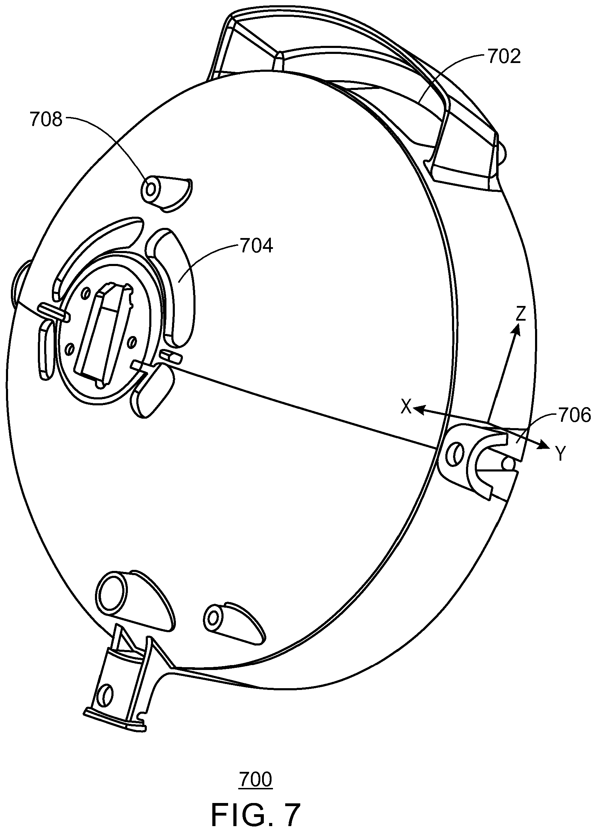

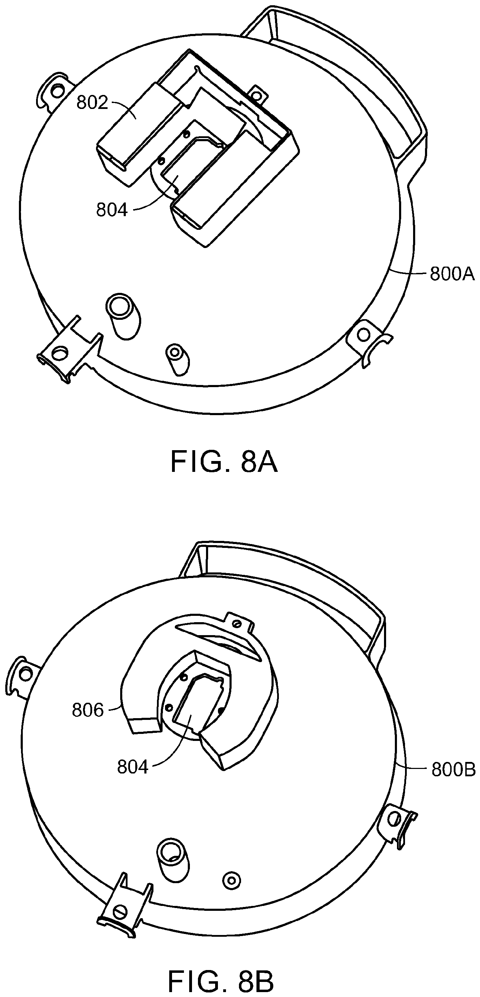

FIG. 6 shows a process flow diagram of an example method for operating a heater with vents for an infant care station in accordance with examples. In some examples, the method 600 can be implemented with any suitable vented heater such as the vented heater 300A and 300B of FIGS. 3A and 3B, 500 of FIG. 5, 700 of FIG. 7, 800A of FIG. 8A, and 800B of FIG. 8B.

At block 602, the method 600 can include determining an expected amount of impurities and sources of impurities to be removed from air. The amount of impurities can be determined based on an amount of irritants, dust, soot, or allergens in the air of an infant care station. The amount of impurities can also be determined based on an amount of impurities from a heater element, which can include impurities emanating from a heater sheath, among others. In some examples, a composition of the impurities can also be determined.

At block 604, the method 600 can include determining a vent configuration for a heater based on the expected amount of impurities. In some examples, the vent configuration can indicate a number of vents to include in a reflective dish, a size of each of the vents, a shape of each of the vents, an orientation of the vents in relation to one another, or the like.

In some examples, each vent can be a different, size, or shape. For example, a vent configuration may indicate that a reflective dish is to include two or more larger vents, two or more smaller vents, among other vents of varying sizes. The vent configuration can indicate a location to place the vents in relation to a center of the reflective dish. By placing the vents proximate to the center of the reflective dish, radiant heat can be projected from the heater to a support platform uniformly without cold or hot spots on the support platform. In some examples, the vents are placed within a predetermined distance from the center of the reflective dish and an outer portion of the reflective dish is a solid material without vents or openings. The outer portion of the reflective dish can include a vent along a perimeter of the reflective dish in some examples.

At block 606, the method 600 can include modifying one or more settings for the heater based on the vent configuration. In some examples, the settings can include a power level, among others. The settings may include, during an installation of a vented heater in an infant care station, a position or shape of a heater element, and geometry of the reflective dish, or the like. For example, the power level can be modified based on a number of vents and/or ducts of a heater. A heater with a larger portion of the reflective dish removed for air flow through vents may operate at a first power level while a heater with a small portion of the reflective dish removed for air flow through vents may operate at a second power level.

At block 608, the method 600 can include providing radiant heat to a support platform using the one or more settings while enabling hot air to flow through vents of the heater. For example, the method 600 can include providing a predetermined amount of radiant heat to a support platform or a bed so that the radiant heat is distributed uniformly across the support platform or bed. As the radiant heat is being projected from a heater element coupled to a reflective dish, hot air can flow through the vents of the reflective dish and away from the infant care station. The vents can enable simultaneously removing impurities from the air around the vented heater while providing uniformly distributed radiant heat to a support platform, bed, and infant patient.

The process flow diagram of method 600 of FIG. 6 is not intended to indicate that all of the operations of blocks 602-608 of the method 600 are to be included in every example. Additionally, the process flow diagram of method 600 of FIG. 6 describes a possible order of executing operations. However, it is to be understood that the operations of the method 600 can be implemented in various orders or sequences. In addition, in some examples, the method 600 can also include fewer or additional operations. In some examples, any number of blocks 602-606 can be performed as part of a calibration and/or installation of a vented heater in an infant care station. For example, blocks 602-608 can be performed as a vented heater is installed in an infant care station as part of manufacturing an infant care station or repairing an infant care station. In some examples, block 608 can be performed as part of the calibration of the vented heater, as well as following installation. For example, providing radiant heat to a support platform using the one or more settings while enabling hot air to flow through vents of the heater can be initially performed as a calibration step before an infant care station is configured for use with infant patients in a care setting. The infant care station, after installation in a care setting, such as a hospital or the like, can use a calibrated vented heater to provide radiant heat to a support platform or infant in real-time using the one or more settings while enabling hot air to flow through vents of the reflective dish of the vented heater.

FIG. 7 shows a back view of an example heater with one or more vents in accordance with examples. In the reflective dish 700 of FIG. 7, there is a vent 702 located along an outer perimeter of the reflective dish 700. There are also central vents 704 located proximate to the center of the reflective dish 700. In some examples, a heater element (not depicted) can mount to the inside of the reflective dish 700 near or at the center of the reflective dish 700. The central vents 704 can enable warm or hot air to flow through to the back of the reflective dish 700 and away from an infant care station. In some examples, the reflective dish 700 can also include any number of features 706 that enable the reflective dish 700 to be coupled to an infant care station. For example, the features 706 enable the reflective dish 700 to be coupled to a canopy of an infant care station, a housing of an infant care station above a support platform, or any other location.

In some examples, the reflective dish 700 can also include any number of duct fasteners 708 that can enable securing a duct to the back of the reflective dish. The ducts are illustrated in FIGS. 8A and 8B and discussed in greater detail below.

FIGS. 8A and 8B depict a back view of example heaters with one or more ducts coupled to vents of the heater in accordance with examples.

As illustrated in FIG. 8A, a reflective dish 800A can include any number of ducts 802 that can couple to the vents 804 of the reflective dish 800A to enable a flow of hot air through the reflective dish 800A and away from an infant care station. In some examples, the hot air can carry or remove any number of impurities from the hot air surrounding the reflective dish 800A to a location away from an infant care station. For example, the ducts 802 can remove or transport hot air with impurities to an exhaust port (not depicted) located on a housing (not depicted) of the infant care station. An exhaust port can enable hot air with impurities to move away from the infant care station without raising an internal temperature or damaging the electronic components, among other components, of the infant care station. The exhaust port can enable impurities from air surrounding the heater of the infant care station to be carried away or removed from an environment of the infant care station. For example, any contaminated air, or the like can be removed from an operating environment of the infant care station while the heater simultaneously provides radiant heat to a support platform or bed.

In some examples, as described above, any number of vents 804 can be included in the reflective dish 800A. For example, a set of vents 804 can be included near or at a center of the reflective dish 800A, a vent 804 may be included at portion of a perimeter of the reflective dish 800A, or a combination thereof. One or more ducts 802 can be coupled to the back of the reflective dish 800A to move, carry, or otherwise transport hot air through the reflective dish 800A and away from an infant care station. The one or more ducts 802 can be included in a housing of the infant care station surrounding the reflective dish 800A of a vented heater. In some examples, the ducts 802 can be contiguous and connect to a single exhaust port in the housing of the infant care station to move hot air away from the operating environment and the electronics of the infant care station. The ducts 802 can also be separately connected to multiple exhaust ports in the housing of the infant care station. For example, a first duct 802 may move hot air from the reflective dish 800A through any number of vents 804 to a first exhaust port and a second duct 802 may move hot air from the reflective dish 800A through any number of vents 804 to a second exhaust port. In some examples, any number of ducts 802 can be used to move hot air with impurities from proximate to a heater element of the reflective dish 800A through the vents 804 and to any number of exhaust ports.

FIG. 8B depicts a reflective dish 800B with vents 804. In the example reflective dish 800B, the ducts 806 can have a different shape than the ducts 802 of FIG. 8A. The ducts 806 can have a different shape to facilitate a different amount of hot air passing through the back of the reflective dish 800B and away from below the reflective dish 800B. The ducts 806 can be configured to receive air from a different number of vents, vents within a different location of a reflective dish, or the like. In some examples, the ducts 806 can be shaped based on an amount of air flow, an amount of impurities in the air, a size of an exhaust port, or the like. As discussed above, the ducts 806 can, in some examples, be split into any number of separate components or parts, which may interface with one or more exhaust ports.

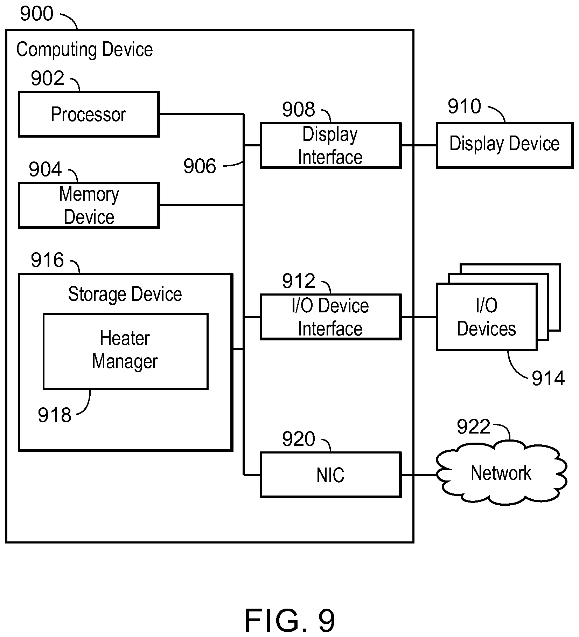

FIG. 9 is a block diagram of an example of a computing device that can operate a heater with vents in an infant care station. The computing device 900 may be, for example, a laptop computer, a desktop computer, a tablet computer, or a mobile phone, among others. The computing device 900 may include a processor 902 that is adapted to execute stored instructions, as well as a memory device 904 that stores instructions that are executable by the processor 902. The processor 902 can be a single core processor, a multi-core processor, a computing cluster, or any number of other configurations. The memory device 904 can include random access memory, read only memory, flash memory, or any other suitable memory systems. The instructions that are executed by the processor 902 may be used to implement a method that can operate a heater with vents in an infant care station, as described in greater detail above in relation to FIGS. 3-8.

The processor 902 may also be linked through the system interconnect 906 (e.g., PCI, PCI-Express, NuBus, etc.) to a display interface 908 adapted to connect the computing device 900 to a display device 910. The display device 910 may include a display screen that is a built-in component of the computing device 900. The display device 910 may also include a computer monitor, television, or projector, among others, which is externally connected to the computing device 900. The display device 910 can include light emitting diodes (LEDs), and micro-LEDs, Organic light emitting diode OLED displays, among others.

The processor 902 may be connected through a system interconnect 906 to an input/output (I/O) device interface 912 adapted to connect the computing device 900 to one or more I/O devices 914 The I/O devices 914 may include, for example, a keyboard and a pointing device, wherein the pointing device may include a touchpad or a touchscreen, among others. The I/O devices 914 may be built-in components of the computing device 900 or may be devices that are externally connected to the computing device 900.

In some embodiments, the processor 902 may also be linked through the system interconnect 906 to a storage device 916 that can include a hard drive, an optical drive, a USB flash drive, an array of drives, or any combinations thereof. In some embodiments, the storage device 916 can include any suitable applications. In some embodiments, the storage device 916 can include a heater manager 918. In some embodiments, the heater manager 918 can operate a heater with vents in an infant care station. For example, during an installation of a vented heater in an infant care station, the heater manager 918 can detect a vent configuration of a heater, wherein the vent configuration indicates a number of vents, a size of the vents, an orientation or position of the vents, and the like. The heater manager 918 can modify a voltage setting, or modify any other settings for the heater based on the vent configuration. In some examples, the heater manager 918 can support multiple heaters with different vent configurations and the heater manager 918 can calibrate each heater separately upon initial installation in an infant care station. The heater manager 918 can manage a temperature variability on the support platform of an infant care station.

In some examples, a network interface controller (also referred to herein as a NIC) 920 may be adapted to connect the computing device 900 through the system interconnect 906 to a network 922. The network 922 may be a cellular network, a radio network, a wide area network (WAN), a local area network (LAN), or the Internet, among others. The network 922 can enable data, such as alerts, among other data, to be transmitted from the computing device 900 to remote computing devices, remote display devices, and the like. In some examples, the heater manager 918 can transmit, using the NIC 920 and the network 922, an alert to any suitable external device such as a mobile device or a computing device, among others.

It is to be understood that the block diagram of FIG. 9 is not intended to indicate that the computing device 900 is to include all of the components shown in FIG. 9. Rather, the computing device 900 can include fewer or additional components not illustrated in FIG. 9 (e.g., additional memory components, embedded controllers, additional modules, additional network interfaces, etc.). Furthermore, any of the functionalities of the heater manager 918 may be partially, or entirely, implemented in hardware and/or in the processor 902. For example, the functionality may be implemented with an application specific integrated circuit, logic implemented in an embedded controller, or in logic implemented in the processor 902, among others. In some embodiments, the functionalities of the heater manager 918 can be implemented with logic, wherein the logic, as referred to herein, can include any suitable hardware (e.g., a processor, among others), software (e.g., an application, among others), firmware, or any suitable combination of hardware, software, and firmware.

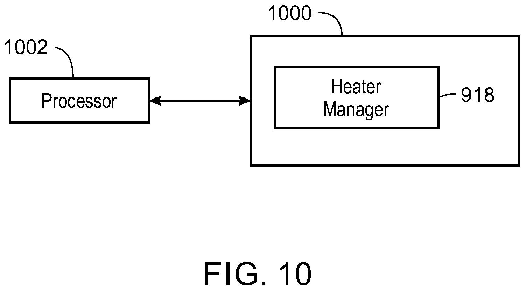

FIG. 10 is an example of a non-transitory machine-readable medium for operating a heater with vents in an infant care station, in accordance with examples. The non-transitory, machine-readable medium 1000 can implement the functionalities of the heater manager 918 of FIG. 9, among others. For example, a processor 1002 can access the non-transitory, machine-readable media 1000.

In some examples, the non-transitory, machine-readable medium 1000 can include instructions to execute a heater manager 918. For example, the non-transitory, machine-readable medium 1000 can include instructions for the heater manager 918 that cause the processor 1002 to operate a heater with vents in an infant care station. In some examples, the heater manager 918 can execute any instructions described above in relation to FIGS. 1-9.

In some examples, the non-transitory, machine-readable medium 1000 can include instructions to implement any combination of the techniques of the heater manager 918 described above.

### EXAMPLES

The disclosure also provides support for an infant care station comprising: a support platform for housing an infant, and a heater for generating radiant heat to be provided to the support platform, wherein a reflective dish of the heater comprises one or more vents to allow hot air proximate to a heater element of the heater to flow through a back of the reflective dish, wherein the hot air comprises impurities, and wherein the heater provides radiant heat to the support platform as the hot air with the impurities flows through the back of the reflective dish. In a first example of the system, a size or a position of the one or more vents are configured to control air flow around the heater element of the heater. In a second example of the system, optionally including the first example, the one or more vents are configured to manage a temperature variability on the support platform. In a third example of the system, optionally including one or both of the first and second examples, the one or more vents create a flow of air behind the reflective dish to at least one duct. In a fourth example of the system, optionally including one or more or each of the first through third examples, the at least one duct transports the flow of air away from the infant care station through an exhaust port in a housing of the infant care station. In a fifth example of the system, optionally including one or more or each of the first through fourth examples, the one or more vents are located within a predetermined distance from a center of the reflective dish. In a sixth example of the system, optionally including one or more or each of the first through fifth examples, a portion of the reflective dish along a perimeter of the reflective dish is a solid surface. In a seventh example of the system, optionally including one or more or each of the first through sixth examples, the reflective dish is configured to provide uniform radiant heat to the support platform.

The disclosure also provides support for a method for operating an infant care station comprising: determining an expected amount of impurities to be removed from air, determining a vent configuration for the heater based on the expected amount of impurities, modifying one or more settings for the heater based on the vent configuration, and providing radiant heat to a support platform of the infant care station using the one or more settings while enabling hot air to flow through one or more vents of the heater. In a first example of the method, the vent configuration comprises a size or a position of the one or more vents that are configured to control air flow around the heater element of the heater. In a second example of the method, optionally including the first example, the one or more vents are configured to manage a temperature variability on the support platform. In a third example of the method, optionally including one or both of the first and second examples, the one or more vents create a flow of air behind a reflective dish of the heater to at least one duct. In a fourth example of the method, optionally including one or more or each of the first through third examples, the at least one duct transports the flow of air away from the infant care station through an exhaust port in a housing of the infant care station. In a fifth example of the method, optionally including one or more or each of the first through fourth examples, the one or more vents are located within a predetermined distance from a center of a reflective dish of the heater. In a sixth example of the method, optionally including one or more or each of the first through fifth examples, the one or more settings comprise settings a power level.

The disclosure also provides support for a non-transitory computer-readable medium for operating an infant care station with a vented heater comprising a plurality of instructions that in response to execution by a processor, cause the processor to: determine an expected amount of impurities to be removed from air, determine a vent configuration for the heater based on the expected amount of impurities, modify one or more settings for the heater based on the vent configuration, and provide radiant heat to a support platform of the infant care station using the one or more settings while enabling hot air to flow through one or more vents of the heater. In a first example of the system, the vent configuration comprises a size or a position of the one or more vents that are configured to control air flow around the heater element of the heater. In a second example of the system, optionally including the first example, the one or more vents are configured to manage a temperature variability on the support platform. In a third example of the system, optionally including one or both of the first and second examples, the one or more vents create a flow of air behind a reflective dish of the heater to at least one duct. In a fourth example of the system, optionally including one or more or each of the first through third examples, the at least one duct transports the flow of air away from the infant care station through an exhaust port in a housing of the infant care station.

Various embodiments may be a system, a method, an apparatus or a computer program product at any possible technical detail level of integration. The computer program product can include a computer readable storage medium (or media) having computer readable program instructions thereon for causing a processor to carry out aspects of various embodiments. The computer readable storage medium can be a tangible device that can retain and store instructions for use by an instruction execution device. The computer readable storage medium can be, for example, but is not limited to, an electronic storage device, a magnetic storage device, an optical storage device, an electromagnetic storage device, a semiconductor storage device, or any suitable combination of the foregoing. A non-exhaustive list of more specific examples of the computer readable storage medium can also include the following: a portable computer diskette, a hard disk, a random access memory (RAM), a read-only memory (ROM), an erasable programmable read-only memory (EPROM or Flash memory), a static random access memory (SRAM), a portable compact disc read-only memory (CD-ROM), a digital versatile disk (DVD), a memory stick, a floppy disk, a mechanically encoded device such as punch-cards or raised structures in a groove having instructions recorded thereon, and any suitable combination of the foregoing. A computer readable storage medium, as used herein, is not to be construed as being transitory signals per se, such as radio waves or other freely propagating electromagnetic waves, electromagnetic waves propagating through a waveguide or other transmission media (e.g., light pulses passing through a fiber-optic cable), or electrical signals transmitted through a wire.

Computer readable program instructions described herein can be downloaded to respective computing/processing devices from a computer readable storage medium or to an external computer or external storage device via a network, for example, the Internet, a local area network, a wide area network or a wireless network. The network can comprise copper transmission cables, optical transmission fibers, wireless transmission, routers, firewalls, switches, gateway computers or edge servers. A network adapter card or network interface in each computing/processing device receives computer readable program instructions from the network and forwards the computer readable program instructions for storage in a computer readable storage medium within the respective computing/processing device. Computer readable program instructions for carrying out operations of various embodiments can be assembler instructions, instruction-set-architecture (ISA) instructions, machine instructions, machine dependent instructions, microcode, firmware instructions, state-setting data, configuration data for integrated circuitry, or either source code or object code written in any combination of one or more programming languages, including an object oriented programming language such as Smalltalk, C++, or the like, and procedural programming languages, such as the "C" programming language or similar programming languages. The computer readable program instructions can execute entirely on the user's computer, partly on the user's computer, as a stand-alone software package, partly on the user's computer and partly on a remote computer or entirely on the remote computer or server. In the latter scenario, the remote computer can be connected to the user's computer through any type of network, including a local area network (LAN) or a wide area network (WAN), or the connection can be made to an external computer (for example, through the Internet using an Internet Service Provider). In some embodiments, electronic circuitry including, for example, programmable logic circuitry, field-programmable gate arrays (FPGA), or programmable logic arrays (PLA) can execute the computer readable program instructions by utilizing state information of the computer readable program instructions to personalize the electronic circuitry, in order to perform various aspects.

Various aspects are described herein with reference to flowchart illustrations or block diagrams of methods, apparatus (systems), and computer program products according to various embodiments. It will be understood that each block of the flowchart illustrations or block diagrams, and combinations of blocks in the flowchart illustrations or block diagrams, can be implemented by computer readable program instructions. These computer readable program instructions can be provided to a processor of a general purpose computer, special purpose computer, or other programmable data processing apparatus to produce a machine, such that the instructions, which execute via the processor of the computer or other programmable data processing apparatus, create means for implementing the functions/acts specified in the flowchart or block diagram block or blocks. These computer readable program instructions can also be stored in a computer readable storage medium that can direct a computer, a programmable data processing apparatus, or other devices to function in a particular manner, such that the computer readable storage medium having instructions stored therein comprises an article of manufacture including instructions which implement aspects of the function/act specified in the flowchart or block diagram block or blocks. The computer readable program instructions can also be loaded onto a computer, other programmable data processing apparatus, or other device to cause a series of operational acts to be performed on the computer, other programmable apparatus or other device to produce a computer implemented process, such that the instructions which execute on the computer, other programmable apparatus, or other device implement the functions/acts specified in the flowchart or block diagram block or blocks.

The flowcharts and block diagrams in the Figures illustrate the architecture, functionality, and operation of possible implementations of systems, methods, and computer program products according to various embodiments. In this regard, each block in the flowchart or block diagrams can represent a module, segment, or portion of instructions, which comprises one or more executable instructions for implementing the specified logical function(s). In some alternative implementations, the functions noted in the blocks can occur out of the order noted in the Figures. For example, two blocks shown in succession can, in fact, be executed substantially concurrently, or the blocks can sometimes be executed in the reverse order, depending upon the functionality involved. It will also be noted that each block of the block diagrams or flowchart illustration, and combinations of blocks in the block diagrams or flowchart illustration, can be implemented by special purpose hardware-based systems that perform the specified functions or acts or carry out combinations of special purpose hardware and computer instructions.

While the subject matter has been described above in the general context of computer-executable instructions of a computer program product that runs on a computer or computers, those skilled in the art will recognize that this disclosure also can or can be implemented in combination with other program modules. Generally, program modules include routines, programs, components, data structures, etc. that perform particular tasks or implement particular abstract data types. Moreover, those skilled in the art will appreciate that various aspects can be practiced with other computer system configurations, including single-processor or multiprocessor computer systems, mini-computing devices, mainframe computers, as well as computers, hand-held computing devices (e.g., PDA, phone), microprocessor-based or programmable consumer or industrial electronics, and the like. The illustrated aspects can also be practiced in distributed computing environments in which tasks are performed by remote processing devices that are linked through a communications network. However, some, if not all aspects of this disclosure can be practiced on stand-alone computers. In a distributed computing environment, program modules can be located in both local and remote memory storage devices.

As used in this application, the terms "component," "system," "platform," "interface," and the like, can refer to or can include a computer-related entity or an entity related to an operational machine with one or more specific functionalities. The entities disclosed herein can be either hardware, a combination of hardware and software, software, or software in execution. For example, a component can be, but is not limited to being, a process running on a processor, a processor, an object, an executable, a thread of execution, a program, or a computer. By way of illustration, both an application running on a server and the server can be a component. One or more components can reside within a process or thread of execution and a component can be localized on one computer or distributed between two or more computers. In another example, respective components can execute from various computer readable media having various data structures stored thereon. The components can communicate via local or remote processes such as in accordance with a signal having one or more data packets (e.g., data from one component interacting with another component in a local system, distributed system, or across a network such as the Internet with other systems via the signal). As another example, a component can be an apparatus with specific functionality provided by mechanical parts operated by electric or electronic circuitry, which is operated by a software or firmware application executed by a processor. In such a case, the processor can be internal or external to the apparatus and can execute at least a part of the software or firmware application. As yet another example, a component can be an apparatus that provides specific functionality through electronic components without mechanical parts, wherein the electronic components can include a processor or other means to execute software or firmware that confers at least in part the functionality of the electronic components. In an aspect, a component can emulate an electronic component via a virtual machine, e.g., within a cloud computing system.

In addition, the term "or" is intended to mean an inclusive "or" rather than an exclusive "or." That is, unless specified otherwise, or clear from context, "X employs A or B" is intended to mean any of the natural inclusive permutations. That is, if X employs A; X employs B; or X employs both A and B, then "X employs A or B" is satisfied under any of the foregoing instances. As used herein, the term "and/or" is intended to have the same meaning as "or." Moreover, articles "a" and "an" as used in the subject specification and annexed drawings should generally be construed to mean "one or more" unless specified otherwise or clear from context to be directed to a singular form. As used herein, the terms "example" or "exemplary" are utilized to mean serving as an example, instance, or illustration. For the avoidance of doubt, the subject matter disclosed herein is not limited by such examples. In addition, any aspect or design described herein as an "example" or "exemplary" is not necessarily to be construed as preferred or advantageous over other aspects or designs, nor is it meant to preclude equivalent exemplary structures and techniques known to those of ordinary skill in the art.

The herein disclosure describes non-limiting examples. For ease of description or explanation, various portions of the herein disclosure utilize the term "each," "every," or "all" when discussing various examples. Such usages of the term "each," "every," or "all" are non-limiting. In other words, when the herein disclosure provides a description that is applied to "each," "every," or "all" of some particular object or component, it should be understood that this is a non-limiting example, and it should be further understood that, in various other examples, it can be the case that such description applies to fewer than "each," "every," or "all" of that particular object or component.

As it is employed in the subject specification, the term "processor" can refer to substantially any computing processing unit or device comprising, but not limited to, single-core processors; single-processors with software multithread execution capability; multi-core processors; multi-core processors with software multithread execution capability; multi-core processors with hardware multithread technology; parallel platforms; and parallel platforms with distributed shared memory. Additionally, a processor can refer to an integrated circuit, an application specific integrated circuit (ASIC), a digital signal processor (DSP), a field programmable gate array (FPGA), a programmable logic controller (PLC), a complex programmable logic device (CPLD), a discrete gate or transistor logic, discrete hardware components, or any combination thereof designed to perform the functions described herein. Further, processors can exploit nano-scale architectures such as, but not limited to, molecular and quantum-dot based transistors, switches and gates, in order to optimize space usage or enhance performance of user equipment. A processor can also be implemented as a combination of computing processing units. In this disclosure, terms such as "store," "storage," "data store," data storage," "database," and substantially any other information storage component relevant to operation and functionality of a component are utilized to refer to "memory components," entities embodied in a "memory," or components comprising a memory. It is to be appreciated that memory or memory components described herein can be either volatile memory or nonvolatile memory, or can include both volatile and nonvolatile memory. By way of illustration, and not limitation, nonvolatile memory can include read only memory (ROM), programmable ROM (PROM), electrically programmable ROM (EPROM), electrically erasable ROM (EEPROM), flash memory, or nonvolatile random access memory (RAM) (e.g., ferroelectric RAM (FeRAM). Volatile memory can include RAM, which can act as external cache memory, for example. By way of illustration and not limitation, RAM is available in many forms such as synchronous RAM (SRAM), dynamic RAM (DRAM), synchronous DRAM (SDRAM), double data rate SDRAM (DDR SDRAM), enhanced SDRAM (ESDRAM), Synchlink DRAM (SLDRAM), direct Rambus RAM (DRRAM), direct Rambus dynamic RAM (DRDRAM), and Rambus dynamic RAM (RDRAM). Additionally, the disclosed memory components of systems or computer-implemented methods herein are intended to include, without being limited to including, these and any other suitable types of memory.

What has been described above include mere examples of systems and computer-implemented methods. It is, of course, not possible to describe every conceivable combination of components or computer-implemented methods for purposes of describing this disclosure, but many further combinations and permutations of this disclosure are possible. Furthermore, to the extent that the terms "includes," "has," "possesses," and the like are used in the detailed description, claims, appendices and drawings such terms are intended to be inclusive in a manner similar to the term "comprising" as "comprising" is interpreted when employed as a transitional word in a claim.

The descriptions of the various embodiments have been presented for purposes of illustration, but are not intended to be exhaustive or limited to the embodiments disclosed. Many modifications and variations will be apparent without departing from the scope and spirit of the described embodiments. The terminology used herein was chosen to best explain the principles of the embodiments, the practical application or technical improvement over technologies found in the marketplace, or to enable others of ordinary skill in the art to understand the embodiments disclosed herein.

## Claims

1. An infant care station comprising:
a support platform for housing an infant; and
a heater for generating radiant heat to be provided to the support platform, wherein a reflective dish of the heater comprises one or more vents to allow hot air proximate to a heater element of the heater to flow through a back of the reflective dish, wherein the hot air comprises impurities, and wherein the heater provides radiant heat to the support platform as the hot air with the impurities flows through the back of the reflective dish.

2. The infant care station of claim 1, wherein a size or a position of the one or more vents are configured to control air flow around the heater element of the heater.

3. The infant care station of claim 1, wherein the one or more vents are configured to manage a temperature variability on the support platform.

4. The infant care station of claim 1, wherein the one or more vents create a flow of air behind the reflective dish to at least one duct.

5. The infant care station of claim 4, wherein the at least one duct transports the flow of air away from the infant care station through an exhaust port in a housing of the infant care station.

6. The infant care station of claim 1, wherein the one or more vents are located within a predetermined distance from a center of the reflective dish.

7. The infant care station of claim 1, wherein a portion of the reflective dish along a perimeter of the reflective dish is a solid surface.

8. The infant care station of claim 1, wherein the reflective dish is configured to provide uniform radiant heat to the support platform.

9. A method for operating an infant care station comprising:
determining an expected amount of impurities to be removed from air;
determining a vent configuration for a heater based on the expected amount of impurities;
modifying one or more settings for the heater based on the vent configuration; and
providing radiant heat to a support platform of the infant care station using the one or more settings while enabling hot air to flow through one or more vents of the heater.

10. The method of claim 9, wherein the vent configuration comprises a size or a position of the one or more vents that are configured to control air flow around a heater element of the heater.

11. The method of claim 10, wherein the one or more vents are configured to manage a temperature variability on the support platform.

12. The method of claim 11, wherein the one or more vents create a flow of air behind a reflective dish of the heater to at least one duct.

13. The method of claim 12, wherein the at least one duct transports the flow of air away from the infant care station through an exhaust port in a housing of the infant care station.

14. The method of claim 9, wherein the one or more vents are located within a predetermined distance from a center of a reflective dish of the heater.

15. The method of claim 9, wherein the one or more settings comprise settings a power level.
